# EUROPEAN PATENT APPLICATION

(11) **EP 3 915 385 A1**
(43) Date of publication of application: **01.12.2021**
(21) Application number: 21175215.9
(22) Date of filing: 21.05.2021
(51) Int. Cl.: A23C 9/142, B01D 61/14, B01D 61/16

(54) **PROCESSING PROTEIN**

(30) Priority: 26.05.2020 AU 2020203454
(71) Applicant: HPS Tech Pty Ltd, Bell Park, Victoria 3215 (AU)
(72) Inventor: Mead, Damien Geoffrey, Bell Park (AU); Kelly, Mark John, Bell Park (AU); Talbot-Walsh, Grace Louise, Bell Park (AU); Kauter, Michael Desmond, Bell Park (AU)
(74) Representative: Håmsø Patentbyrå AS

(57) **Abstract**

A method comprising feeding MF feedstock to a cross-flow microfilter. The MF feedstock comprises at least 5% protein solids and has a conductivity of less than 10 mS/cm.

## Description

### FIELD

The invention relates to processing protein.

The invention will be described with reference to processing dairy proteins by way of example only. Variants of the concepts disclosed herein may be applied in connection with other proteins.

### BACKGROUND

Protein is often found in material carrying a bacterial load. Pasteurisation is one option for cleaning a protein stream, although some pathogens are heat resistant. Microfiltration through lenticular filters is another option for cleaning a protein stream.

Lenticular filters have a limited lifespan and must be regularly changed. Even with frequent planned changes, blockages can and do occur unexpectedly from time to time. Filter changes are costly. In a commercial-scale plant processing a single batch of lactoferrin per week, four filter changes might be expected per week. Each filter change may well entail discarding a filter embedded with about 1 kg of lactoferrin, corresponding to a loss in the vicinity of 208 kg of lactoferrin per year. At the time of writing, lactoferrin was retailing at about US$1,000 per kg, whereby the plant's filtration system costs in the vicinity of US$200,000 per year simply in lost lactoferrin, before accounting for the cost of the filters per se, and the labour associated with the filter changes, etc.

With the foregoing in mind, the present invention aims to provide improvements in and for processing protein, or at least to provide an alternative for those concerned with processing protein.

It is not admitted that any of the information in this patent specification is common general knowledge, or that the person skilled in the art could be reasonably expected to ascertain or understand it, regard it as relevant or combine it in any way before the priority date.

### SUMMARY

One aspect of the invention provides a method comprising feeding MF feedstock to a cross-flow microfilter;
the MF feedstock comprising at least 5% protein solids and having a conductivity of less than 10 mS/cm.

'MF feedstock' is used herein simply as a label for the material fed to the cross-flow microfilter. Terminology such as '% protein solids' and '% total solids' as used herein refer to percentages calculated on a mass per unit volume basis, as is conventional in the context of processing proteins.

Preferably, the MF feedstock has at least 10%, e.g. at least 13%, protein solids. The conductivity is preferably less than 5 mS/cm, e.g. less than 3 mS/cm. The feeding the MF feedstock is preferably at a temperature of at least (or preferably more than) 5°C, e.g. in the range of 10°C to 70°C (inclusive) such as in the range 10°C to 50°C (inclusive), or more preferably in the range of 20°C to 40°C (inclusive).

The cross-flow microfilter may have a cross-flow velocity of more than 3 m/sec, e.g. at least 6 m/sec. Preferably, the cross-flow velocity is not more than 10 m/sec, e.g. not more than 7 m/sec.

The method may comprise recirculating retentate from the cross-flow microfilter.

The method may comprise producing the MF feedstock. Preferably, the producing the MF feedstock comprises one or both of concentrating, and removing salt from, a precursor. The producing the MF feedstock may comprise ultrafiltration. The producing the MF feedstock may comprise diafiltration.

The method may comprise producing the precursor. The producing the precursor may comprise elution of an input material. The input material may be a dairy product.

The protein solids may be dairy protein solids.

Another aspect of the invention provides an apparatus comprising
a system to concentrate and remove salt from a protein solution to produce an intermediary; and
a microfiltration system to remove bacteria from the intermediary;
wherein the microfiltration system comprises a cross-flow microfilter.

The apparatus may comprise a temperature regulator to regulate a temperature of an MF feedstock fed to the cross-flow microfilter. The microfilter preferably has a pore size in the range of 0.8 µm to 2 µm (inclusive). The apparatus may comprise a loop for recirculating retentate from the microfilter. Preferred variants comprise a flow regulation system configured to maintain a cross-flow velocity in the range of 3 m/sec to 10 m/sec (inclusive). The apparatus may comprise an elution system to produce the protein solution.

### BRIEF DESCRIPTION OF DRAWINGS

Figure 1 illustrates a method of producing lactoferrin powder;
Figure 2 schematically illustrates a microfiltration system; and
Figure 3 charts viscosity as a function of temperature.

### DESCRIPTION OF EMBODIMENTS

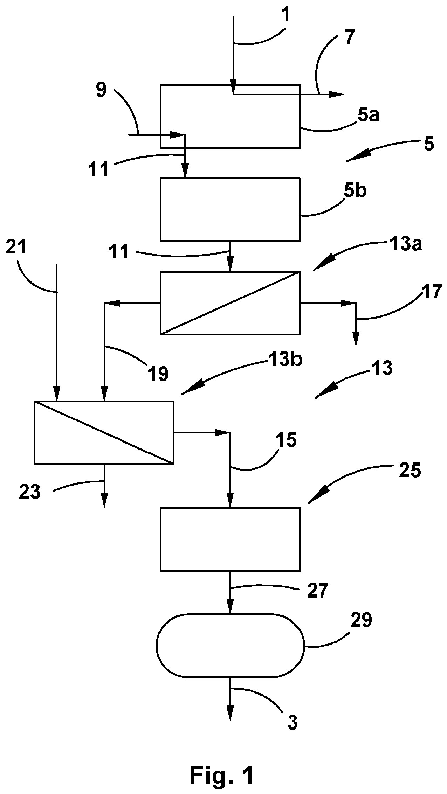

Figure 1 illustrates a method and apparatus for processing a protein solution to produce a clean concentrate, or more specifically for processing skim milk 1 to produce clean lactoferrin powder 3 in this case. Lactoferrin is a valuable protein useful in products such as baby formula. It is present in skim milk in very low concentrations (typically 100 to 300 mg lactoferrin/litre skim milk).

Skim milk 1 is fed to an elution system 5. The system 5 comprises an ion exchange column 5a and a storage tank 5b. Within the column 5a, ion exchange chromatography is employed to separate the target proteins from the skim milk 1. The column 5a comprises ion exchange resin to which the majority of the target protein (lactoferrin in this case) selectively binds.

Resin sold by CYTIVA™ under the trade mark SP SEPHAROSE BIG BEADS is preferred for recovering lactoferrin. Other strong cation resins may be used, e.g. SEPRAFLO S™ (SEPRAGEN CORPORATION™) and those suited to expanded bed chromatography such as FASTLINE SP™ (UPFRONT CHROMATOGRAPHY™) and STREAMLINE DIRECT HST™ (GE HEALTHCARE BIOSCIENCES™).

After loading the resin with the skim milk, the milk flow is stopped, and the resin is rinsed with water. This displaces protein-depleted skim milk from the resin bed to produce a stream 7 that can be recovered for use in other products.

A salt-based eluent 9 is then supplied to wash the protein from the column 5a to produce eluant 11 which is captured and stored within the tank 5b. The eluant 11 preferably comprises sodium chloride although there are other options. By way of example, the buffer salt might be disodium phosphate, monosodium phosphate, potassium phosphate, sodium hydroxide, sodium acetate, acetic acid or potassium chloride.

Preferably, the elution takes place in 2 steps. Firstly, there is a low conductivity elution (∼ 30-60 mS/cm) step using relatively dilute (0.3-0.75 molar) sodium chloride solution. This is essentially a second rinsing step and its output is also directed to the stream 7. This second rinsing step removes most of the loosely bound proteins, e.g. non-target proteins such as lactoperoxidase, to ensure the high purity of the target protein remaining bound to the resin. The stream 7 may be recovered for further processing or can be discarded.

Secondly, elution with a strong sodium chloride solution (approximately 1 molar), removes most protein molecules remaining on the ion exchange resin to produce the eluant 11. Then the resin is cleaned or regenerated and is readied for processing the next batch of skim milk. The eluant 11 is a dilute, salty protein solution having a concentration of about 5 Brix and is the precursor to a system 13.

'Brix' (sometimes written as °Bx) is a measure of concentration that is easily measured in a factory environment. Typically, a hand-held refractometer is used. The refractometer in fact measures refractive index but displays a result in Brix. Brix is the percentage of total solids in a sucrose solution having that refractive index.

Through empirical testing, the inventors have found that, for pure lactoferrin solution, Brix = 0.8276 (total solids) + 0.0273 (with an R² value in excess of 0.99). The inventors have found that the same calibration is a useful approximation in the context of solutions that contain both protein and salt.

In a preferred implementation, plural batches of eluant are stored in a holding tank (not shown) before being passed on to the system 13. In the illustrated variant, each batch produces about 6,000 L of eluant 11, and typically three to six batches (about 18,000 to about 36,000 L) are sent to the system 13 as a single composite batch.

The system 13 serves to concentrate, and remove the salt from, the precursor to produce intermediary 15. Dialysis, electrolysis and gel filtration chromatography are other potential means of concentrating protein and removing salt. The system 13 comprises an ultrafiltration system 13a that serves to concentrate the eluant 11, and a diafiltration system 13b that removes salt from the concentrated eluant. In this example, the system 13 concentrates the eluant 11 by a factor of about 36, whereby the about 18,000 to about 36,000 L is reduced to about 500 L to about 1,000 L.

The ultrafiltration system 13a comprises a membrane having a molecular weight cut-off of 30 kDa. Salt and water permeate through the membrane to concentrate proteins larger than this weight cut-off in the retentate 19. The concentration of protein in retentate 19 is in the range of about 10 to about 25 Brix, or more preferably in the range of 20 to 25 Brix. This rententate retains some salt and may well have a conductivity in the range of about 80 to about 90 mS/cm). Permeate 17 from the ultrafiltration system 13a is a waste stream from which salt may be recovered and reused.

The retentate 19 is supplied to the diafiltration system 13b along with dilutant (e.g. water) 21 to remove salt whilst maintaining protein concentration. The intermediary 15 is the retentate from the diafiltration system 13b and has a protein concentration in the range of 10 to 25 Brix, or more preferably in the range of 20 to 25 Brix, and conductivity in the range of 1 to 3 mS/cm. The salt-laden permeate 23 from the diafiltration system 13b is another waste stream from which salt may be recovered and reused.

In this example, the diafiltration system 13b removes salt whilst maintaining protein concentration. There are other options. By way of example, the ultrafiltration system 13a may be configured to produce retentate having a protein concentration of about 33 Brix and the diafiltration system 13b may be configured to dilute this material; and vice versa the diafiltration system 13b may be employed to concentrate the protein.

The intermediary 15 is supplied to a microfiltration system 25 to be cleansed of bacteria to produce cleansed stream 27. Stream 27 is then subject to downstream processing 29 comprising drying to produce the lactoferrin powder 3. The powder preferably has an "Ash" specification which is <1.0% (or <0.6% Na).

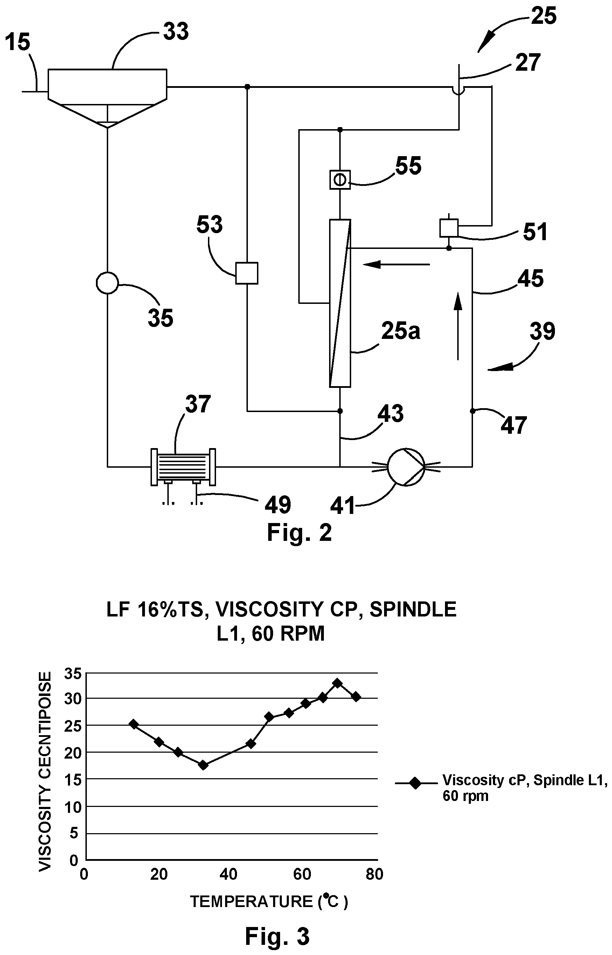

The microfiltration system 25 comprises a cross-flow microfilter 25a and is illustrated in more detail in Figure 2.

The microfiltration system 25 further comprises a collection tank 33 to receive the intermediary 15. A supply pump 35 pumps fluid from the tank 33 through a heater 37 en route to a recirculation loop 39. A loop pump 41 pumps fluid about the loop 39. The microfilter 25a sits within the loop 39. Retentate 43 from the filter 25a is mixed with the fluid from the tank 33 to form the MF feedstock 45 that is supplied to the microfilter 25a. A portion of the retentate 43 is recirculated through the tank 33 via the normally open valve 53. By separately controlling the pumps 35, 41 the feed pressure to, and the cross flow velocity through, the microfilter 25a can be controlled.

The present inventors have recognised that by adopting a cross-flow microfilter to cleanse the product stream, the cost associated with blocked dead-end filters can be avoided.

The precursor 11 has a lower viscosity than the intermediary 15. The change in viscosity is partly attributable to the removal of salt. The inventors have observed that even small reductions in salt content are correlated with higher pressures and reduced microfiltration flux rate. A negative relationship between viscosity and salt concentration is also observed in Mela et al 'Charge reversal by salt-induced aggregation in aqueous lactoferrin solutions' Colloids and Surfaces B: Biointerfaces, Volume 78, Issue 1, June 2010, 53-60 (https://doi.org/10.1016/j.colsurfb.2010.02.011)

Since the precursor 11 has a lower viscosity than the intermediary 15, it may well be thought of as a better candidate for microfiltration. However, the inventors have recognised that by filtering the more concentrated stream 15, a much smaller and less expensive microfiltration system can be used, and that by carefully selecting processing parameters, fouling of the cross-flow filter can be avoided (or at least very much reduced). Furthermore, cleansing the stream 11 would leave the potential for the cleansed stream to be sullied by the subsequent concentration step.

Proteins are typically processed at about 4°C to 5°C to retard the growth rate of bacteria. The present inventors have recognised that protein streams at higher temperatures, say 10°C or more, have lower viscosities. By heating a protein concentrate akin to the intermediary 15 from 5°C to 10°C, viscosity can be reduced to a value better suited to cross-flow microfiltration. There is a practical limit to the temperature range. As temperatures are elevated beyond about 50°C, various proteins lose functionality and denature at different temperatures. By way of example, egg white in a frying pan will start to denature at close to 57°C and whey proteins denature at about 70°C. The risk of problems such as microbial growth and gelling also increases with temperature.

Figure 3 charts the viscosity profile of an example of the intermediary 15 in the form of a lactoferrin concentrate comprising 16% total solids and minimal salt content corresponding to a conductivity of about 2 mS/cm. As illustrated, there is a local minima in the vicinity of 30°C, thus the MF feedstock 45 is preferably at a temperature of about 30°C. The temperature is preferably in the range of 28°C to 32°C, such as at 28°).

Other proteins have similar viscosity profiles. A literature search shows an example of concentrated protein solutions where viscosity decreases with increasing temperature. These studies typically consider temperatures in the range of about 5°C through to something in the vicinity of 50°C, see for example:
- Monkos, K 'Concentration and temperature dependence of viscosity in lysozyme aqueous solutions' Biochimica et Biophysica Acta (BBA) - Protein Structure and Molecular Enzymology, Volume 1339, Issue 2, May 1997, 304-310 https://doi.org/10.1016/S0167-4838(97)00013-7
- Monkos, K 'Viscosity of bovine serum albumin aqueous solutions as a function of temperature and concentration' International Journal of Biological Macromolecules, Volume 18, Issues 1-2, February 1996, 61-68 https://www.ncbi.nlm.nih.gov/pubmed/8852754
- Monkos, K 'Viscometric study of human, bovine, equine and ovine haemoglobin in aqueous solution' International Journal of Biological Macromolecules, Vol 16, Issue 1, February 1994, 31-35 https://www.sciencedirect.com/science/article/pii/0141813094900086

To maintain the MF feedstock temperature, the microfiltration system 25 comprises a temperature regulator 37, 47 comprising a thermal device 37 and a temperature sensor 47 in response to which the thermal device 37 is controlled.

Whilst in principle the thermal device might take the form of a cooling device, e.g. in the context of a hotter upstream process, in this case it is a heater. More specifically, it is a heat exchanger, although other forms of heater are possible. The heat exchanger is a counter-flow heat exchanger supplied with water 49 at 35°C. The temperature regulator 37, 47 comprises a control arrangement by which the flow rate of the water 49 is varied in response to the temperature sensor 47. Of course, other forms of temperature regulation are possible, and the temperature of the MF feedstock 45 might be regulated without its temperature being directly measured. By way of example, the temperature of the MF feedstock might be inferred from a temperature of the permeate 27. The water 49 might be replaced by any other convenient fluid.

Cross-flow microfilters are frequently operated at cross-flow velocities in the vicinity 1 to 3 m/sec. The present inventors have found that by operating the filter at higher cross-flow velocities, the risk/frequency of fouling can be reduced. Preferably, the cross-flow velocity is in the range of 6 to 7 m/sec.

Microfiltration can entail membrane pore sizes in the range of 0.1 µm to 10 µm. The inventors have found pore sizes in the range of 0.8 µm to 2 µm to be an advantageous compromise between permeation rate (flux) on the one hand, and pore blockage/membrane fouling and effective bacteria removal on the other hand.

The membrane 25a may be either organic or inorganic. Organic membranes are inexpensive and offer very large surface areas. On the other hand, inorganic (e.g. ceramic) commercial-scale membranes having appropriate pore sizes are more easily sourced. Relative to conventional polyethersulfone membranes, ceramic membranes have higher thermal and chemical stability, higher resistance to back pressure, and a longer service life, e.g. an expected lifespan in excess of five years before replacement. The inventors have found that a TAMI ISOFLUX™ ceramic membrane having a pore size of 1.4 µm is a good choice, particularly in the context of lactoferrin protein that has a 77 to 80 kDa molecular weight.

Testing has shown that variants of the microfiltration unit 25 can operate without concentrating protein. By way of example, an advantageous embodiment of the microfiltration unit 25 is configured to deliver cleansed concentrate 27 at a rate of 180 L/hr and has a microfilter 25a in the form of an INSIDE CERAM™ filtration unit supplied by TAMI INDUSTRIES™. This unit comprises 55 individual TAMI ISOFLUX™ ceramic membranes in one SS316 housing and is characterised as follows:

| | |
|---|---|
| Number of Membranes | 55 |
| Membrane Configuration | 8 channel membrane at φ25 |
| Housing Certification | USDA |
| Filtration Area | 11 m² |
| Cross-flow Rate | 6-7 m/s |

This embodiment of the microfiltration unit 25 was tested in three separate tests with intermediaries 15 comprising 16, 20 and 22 Brix lactoferrin concentrations. The shortest of these tests lasted 50 minutes. Each test showed once steady state was reached the concentrations of intermediary 15, permeate 27 and retentate 43 remained substantially equal to each other, e.g. there was no observable accumulation of protein in the retentate. The testing also showed more than 99.9% reduction in the amount of the bacteria. Preferably, intermediary 15 has a protein concentration of not more than 33%, or preferably not more than 25 Brix. This embodiment of the microfiltration unit 25 has been tested at about 24 Brix and shown to work well, although the inventors believe that higher concentrations may well lead to reduced permeation rates.

Optionally, a holding tank (not shown) sits between the system 13 and the microfiltration system 25 to hold the intermediary 15 until the downstream processes 29 are ready to accept product. The intermediary 15 is kept cool (e.g. by recirculation though a heat exchanger at a set point of 8°C). When all flag conditions have been met, intermediary 15 is transferred from the holding tank to the MF feed tank 33. From this point, bleed valve 51 is temporarily opened to remove air whilst the loop 39 is primed, and the loop pump started to increase the transmembrane pressure and ensure permeation. Preferably, the pump 35 is controlled to hold the retentate 43 at a baseline pressure of 0.8 bar, the pump 41 is controlled to drive the MF feedstock 45 to a feed pressure of 1.5 bar, and downstream restrictions are controlled to maintain a transmembrane pressure in the range of 0.5 to 0.4 bar. Higher transmembrane pressures are possible, although preferably the transmembrane pressure is kept below 2.5 bar to protect the membrane from damage.

The microfiltration system 25 can continue to run as the upstream processes are flushed with water (or other liquid) and in turn the protein concentration of the intermediary 15 drops. Preferably, the microfiltration system 25 continues to operate in this way until the protein concentration of the intermediary 15 (and in turn the protein concentration of the cleansed stream 27) drops below a threshold. Most preferably, at the end of a production run (e.g. when the volume of fluid within the tank 33 falls below a threshold), water (or other dilutant) is introduced into the tank 33 to push substantially all of the remaining lactoferrin through the microfiltration system. At about the same time as water is supplied to the tank 33 (and/or when a protein concentration of the MF feedstock 45 falls below a threshold), the permeate may be redirected to the recovery tank (e.g. a 1200±500 L tank) until that tank is full.

The microfiltration system 25 can then be stopped so the bacteria-laden retentate 43 can be dumped and so the system 25 can be cleaned in place (CIP). The normally open valve 55 is pulsed during CIP.

Figure 1 illustrates a process having skim milk as its input material. Upstream of the illustrated process steps, whole milk is separated into skim milk and cream. Preferably the skim milk fat concentration is no higher than 0.1% m/v to avoid eventual blocking of the ion-exchange column. Skim milk quality can be improved by passing the skim through a centrifugal clarifier to remove bacterial spores.

Other input materials, such as other dairy products, are possible. Variants of the method and apparatus may be applied to input materials such as defatted cheese whey, native whey, blood serum, plant-based protein solutions (e.g. cereals, legumes, oilseeds, ancient grains, algal, fungal, yeast, and hemp) and egg protein solutions. Dissolution may be called for in connection with solid input materials.

Figure 1 illustrates a process in which elution is employed to produce precursor 11 (dilute, salty lactoferrin solution). Alternatively, variants of the system 13 and microfiltration system 25 may be employed to process other precursors, e.g. for the purification of other dairy proteins (such as α-lactalbumin, β-lactoglobulin, bovine serum albumin (BSA), immunoglobulins (A, M and C), glycomacropeptides, lactoperoxidase and osteopontin) or proteins isolated from animal-based proteins, blood products, bio-tech streams, or plant-based raw materials. The precursor to systems 13, 25 is preferably an eluent although other precursors are contemplated.

Figure 1 illustrates a process in which the microfiltration system 25 is employed to cleanse a protein concentrate in the form of the intermediary 15. Variants of the microfiltration system 25 may be usefully employed to process other protein concentrates, e.g. powdered protein may be dissolved and cleansed via microfiltration.

The invention is not limited to the examples disclosed herein. Rather, the invention is defined by the claims.

The term 'comprises' and its grammatical variants has a meaning that is determined by the context in which it appears. Accordingly, the term should not be interpreted exhaustively unless the context dictates so.

## Claims

1. A method comprising feeding MF feedstock to a cross-flow microfilter;
the MF feedstock comprising at least 5% protein solids and having a conductivity of less than 10 mS/cm.

2. The method of claim 1 wherein the MF feedstock has at least 13% protein solids.

3. The method of claim 1 or 2 wherein the conductivity is less than 3 mS/cm.

4. The method of claim 1, 2 or 3 wherein the feeding the MF feedstock is at a temperature in the range of 10°C to 50°C (inclusive).

5. The method of claim 1, 2 or 3 wherein the feeding the MF feedstock is at a temperature in the range of 20°C to 40°C (inclusive).

6. The method of any one of claims 1 to 5 wherein the cross-flow microfilter has a cross-flow velocity of more than 3 m/sec.

7. The method of any one of claims 1 to 5 wherein the cross-flow microfilter has a cross-flow velocity of not more than 10 m/sec.

8. The method of any one of claims 1 to 7 comprising recirculating retentate from the cross-flow microfilter.

9. The method of any one of claims 1 to 8 comprising producing the MF feedstock;
the producing the MF feedstock comprising one or both of concentrating, and removing salt from, a precursor.

10. The method of claim 9 wherein the producing the MF feedstock comprises ultrafiltration.

11. The method of claim 9 or 10 wherein the producing the MF feedstock comprises diafiltration.

12. The method of claim 9, 10 or 11 comprising producing the precursor;
the producing the precursor comprising elution of an input material.

13. The method of claim 12 wherein the input material is a dairy product.

14. The method of any one of claims 1 to 12 wherein the protein solids are dairy protein solids.

15. An apparatus comprising
a system to concentrate and remove salt from a protein solution to produce an intermediary; and
a microfiltration system to remove bacteria from the intermediary;
wherein the microfiltration system comprises a cross-flow microfilter; and
the apparatus is configured to feed to the cross-flow microfilter MF feedstock comprising at least 5% protein solids and having a conductivity of less than 10 mS/cm.
